# EUROPÄISCHE PATENTSCHRIFT

(11) **EP 2 839 846 B1**
(45) Veröffentlichungstag und Bekanntmachung des Hinweises auf die Patenterteilung: **13.05.2020**
(21) Anmeldenummer: 14181946.6
(22) Anmeldetag: 22.08.2014
(51) Int. Cl.: A61L 2/22

(54) **VORRICHTUNG ZUR BEHANDLUNG VON BEHÄLTERN IN EINER GETRÄNKEABFÜLLANLAGE**
DEVICE FOR TREATMENT OF CONTAINERS IN A BEVERAGE BOTTLING SYSTEM
DISPOSITIF DE TRAITEMENT DE RÉCIPIENTS DANS UNE INSTALLATION DE REMPLISSAGE DE BOISSONS

(30) Priorität: 22.08.2013 DE 102013109089
(43) Veröffentlichungstag der Anmeldung: 25.02.2015
(73) Patentinhaber: Krones AG, 93073 Neutraubling (DE)
(72) Erfinder: Müller, Holger, 93073 Neutraubling (DE); Grünwald, Angela, 93073 Neutraubling (DE); Söllner, Jürgen, 93073 Neutraubling (DE)
(74) Vertreter: Nordmeyer, Philipp Werner

(56) Entgegenhaltungen:
- EP-A1- 2 722 089
- EP-A2- 0 410 306
- WO-A1-00/29038
- WO-A1-03/070024
- DE-A1-102010 002 909
- DE-A1-102010 003 468
- DE-U1- 9 306 797

## Beschreibung

### Technisches Gebiet

Die vorliegende Erfindung betrifft eine Vorrichtung zur Behandlung von Behältern in Getränkeabfüllanlagen, bevorzugt zur Desinfektion von Behältern in einer Getränkeabfüllanlage, beispielsweise in einer Nass-Aseptik-Anlage.

### Stand der Technik

In Getränkeabfüllanlagen ist es bekannt, die Behandlung der zu befüllenden Behälter, beispielsweise eine Desinfektion, eine Spülung und/oder eine Abfüllung des Füllproduktes, innerhalb so genannter Isolatoren durchzuführen, um die Behälterbehandlung unter definierten Umgebungsbedingungen durchführen zu können.

Ein solcher Isolator schirmt den darin definierten Isolatorraum gegenüber der Umgebung ab, so dass es möglich ist, im Isolatorraum eine keimarme und/oder sporenarme beziehungsweise keimfreie und/oder sporenfreie Atmosphäre zu schaffen, wodurch die Behälterreinigung, Behältersterilisierung, Befüllung mit dem Füllprodukt und/oder das Verschließen der Behälter so durchgeführt werden kann, dass eine möglichst geringe Belastung der Behälter sowie des Füllproduktes mit Keimen und Sporen stattfindet. Auf diese Weise kann die Qualität des Füllproduktes über einen längeren Zeitraum hinweg aufrechterhalten werden und die mögliche Lagerdauer für das jeweilige Füllprodukt kann erhöht werden.

In modernen Getränkeabfüllanlagen durchläuft ein zu befüllender Behälter dabei unterschiedliche Stationen, wobei beispielsweise bei einer Abfüllung des Füllprodukts in eine Einweg-Kunststoffflasche die Kunststoffflasche vor der Befüllung mit einem flüssigen Desinfektionsmittel ausgespült wird und danach mit Sterilwasser klargespült wird. Zur Herstellung des verwendeten Desinfektionsmittels wird üblicherweise in einem ersten Reservoir ein Desinfektionsmittelkonzentrat mit Prozesswasser ausgemischt, temperiert und zur Behälterdesinfektion auf die entsprechenden Behälteroberflächen aufgebracht. Das von den behandelten Behälteroberflächen wieder abfließende Desinfektionsmittel wird üblicherweise im Kreislauf geführt, wobei es in dem ersten Reservoir gesammelt und vorgehalten wird, in welchem üblicherweise auch eine Konzentrationskontrolle sowie eine Temperierungskontrolle durchgeführt wird.

Nach der Behandlung mit dem Desinfektionsmittel werden die Behälter mittels frisch zugeführtem Sterilwasser gespült und das abfließende Spülwasser dann in einem zweiten Reservoir zur weiteren Verwendung vorgehalten.

Das Desinfektionsmittel in dem ersten Reservoir muss gegenüber der Umgebung abgeschlossen sein, um ein Entweichen von schädlichen Gase, beispielsweise Chlor- oder Peroxydgasen, zu vermeiden. Daher ist das erste Reservoir üblicherweise mit einem Deckel verschlossen, welcher mittels eines Schutzschalters verriegelt ist. Der Schutzschalter ist Bestandteil des Sicherheitskreises der gesamten Getränkeabfüllanlage, so dass bei einem Öffnen des Deckels oder bei einem Defekt oder einer Unterbrechung des Schutzschalters die gesamte Getränkeabfüllanlage zum Stillstand kommt. Weiterhin ist die hermetische Abdichtung des Reservoirs gegenüber der Umgebung notwendig, um eine Geruchsbelastung sowie eine Belastung der Anlagenbediener mit Desinfektionsmitteldämpfen zu vermeiden.

Die Reservoirs für das Desinfektionsmittel beziehungsweise für das Spülwasser sind gemäß dem Stand der Technik beabstandet von dem jeweiligen Isolator angeordnet und beispielsweise über Rohrleitungen mit den entsprechenden Zulauf- und Ablaufvorrichtungen des Isolators verbunden. Durch diese Rohrleitungen ergibt sich jedoch eine Verzögerung in der möglichen Steuerung der Temperierung der Behandlungsmedien beziehungsweise der Konzentration des Desinfektionsmediums, da sich das in der Leitung befindliche Desinfektionsmittelvolumen nicht mehr beeinflussen lässt. Entsprechend kann es vorkommen, dass das sich im Rohrleitungssystem befindliche Desinfektionsmittel nicht die ausreichende Konzentration für die Behälterdesinfektion aufweist.

Eine Nass-Aseptik-Anlage ist beispielsweise aus der DE 10 2010 054 788 A1 bekannt. Eine Vorrichtung zur thermischen Behandlung von Produkten mit Reinigung der Prozessflüssigkeit geht aus der EP 2 722 089 A1 hervor. Die DE 93 06 797 U1 beschreibt ein Becherfüllwerk für dünnflüssige bis pastöse Molkerei- und Fettprodukte. Die DE 10 2010 003 468 A1 und DE 10 2010 002 909 A1 beschreiben Vorrichtungen zum Sterilisieren von becherförmigen Verpackungsbehältern. Die WO 00/29038 A1 betrifft die Reinigung von Behältern.

### Darstellung der Erfindung

Ausgehend von dem bekannten Stand der Technik ist es eine Aufgabe der vorliegenden Erfindung, eine Vorrichtung zur Behandlung von Behältern in einer Getränkeabfüllanlage anzugeben, welche eine weiter verbesserte Behandlungsqualität ermöglicht.

Entsprechend wird eine Vorrichtung zur Behandlung von Behältern in einer Getränkeabfüllanlage, erfindungsgemäß zur Desinfektion von Behältern in einer Getränkeabfüllanlage, umfassend einen gegenüber der Umgebung abgeschirmten Isolatorraum, in welchem eine Behälterbehandlung durchführbar ist, und ein Reservoir zur Aufnahme eines Behandlungsfluids, das ein Desinfektionsmittel ist, zur Behandlung der Behälter vorgeschlagen. Erfindungsgemäß ist das Reservoir in den Isolatorraum integriert.

Erfindungsgemäß ist die Behälterbehandlungsvorrichtung so vorgesehen, dass sie das Behandlungsfluid in einer unteren Ebene des Reservoirs abzieht und dieses dann bevorzugt unter Druck und bevorzugt temperiert über eine Behälterbehandlungsdüse auf die zu behandelnden Behälter aufbringt. Mittels einer solchen Behälterbehandlungsvorrichtung kann eine zuverlässige Behälterbehandlung im Isolatorraum durchgeführt werden.

Dadurch, dass das Reservoir in dem Isolatorraum integriert ist, kann auf eine aufwändige und ausgedehnte Verrohrung des Reservoirs mit den im Isolatorraum vorgesehenen Behandlungsvorrichtungen verzichtet werden und damit die mit den in den jeweiligen Rohrleitungen einhergehenden und schwer kontrollierbaren Behandlungsfluidvolumina reduziert werden. So können die gemäß dem Stand der Technik entstehenden Verzögerungen reduziert beziehungsweise vollständig vermieden werden und es ergibt sich eine unmittelbarere Steuerung beispielsweise der Desinfektionsmittelkonzentration in einem Desinfektionsfluid beziehungsweise eine genauere Temperierung kann erreicht werden.

Darüber hinaus kann auf zusätzliche Sicherheitsvorrichtungen zur Überwachung des jeweiligen Reservoirs verzichtet werden, da die bereits im Isolatorraum vorhandenen Sicherheitsvorrichtungen für das Reservoir genutzt werden können. Entsprechend kann die Betriebssicherheit erhöht werden, da aufgrund einer reduzierten Anzahl von Sicherheitsvorrichtungen die Gesamtausfallwahrscheinlichkeit verringert wird.

Durch die Integration des Reservoirs in den Isolatorraum kann weiterhin eine Geruchsbelastung beziehungsweise das Auftreten von erhöhten Desinfektionsmittelkonzentrationen im Außenbereich des Isolators beziehungsweise der Getränkeabfüllanlage und besonders auch in den Arbeitsbereichen der jeweiligen Anlagenbediener vermieden werden, da ein Ausgasen beziehungsweise Übertreten von Desinfektionsmittel aus dem Reservoir in die Umgebung nicht stattfinden kann. Eventuelle Belastungen können nur in den nach außen hin abgedichteten Isolator einströmen. Entsprechend ist die Betriebssicherheit auch hier dahingehend erhöht, dass keine zusätzliche Quelle für die Emissionen von Geruchsbelastungen beziehungsweise Schadstoffbelastungen vorliegt.

Weiterhin vorteilhaft an der Integration des Reservoirs in dem Isolatorraum ist, dass im Isolatorraum bereits vorhandene Reinigungsvorrichtungen zur Reinigung des Isolatorraumes, wie beispielsweise Reinigungsdüsen zum Aufbringen von Reinigungsfluiden zur Reinigung der Innenflächen der Wände des Isolators, auch zur Reinigung des Reservoirs verwendet werden können. Auch dadurch wird die Gesamtvorrichtung weniger komplex, da zusätzliche beziehungsweise separate Reinigungsvorrichtungen zur automatisierten Reinigung des Reservoirs nicht mehr notwendig sind.

Besonders vorteilhaft besteht eine Fluidverbindung zwischen dem Reservoir und den Reinigungsvorrichtungen, so dass auch während eines Reinigungsbetriebes ein Behandlungsfluid in dem Reservoir gesammelt wird. In einem Reinigungskreislauf ist folglich kein zusätzliches Reservoir notwendig.

Bevorzugt bildet dabei ein Teil des Isolatorraums das Reservoir aus. Der Isolatorraum ist bevorzugt nach unten hin durch einen Isolatortisch beziehungsweise einen Isolatorboden begrenzt, und das Reservoir ist im Isolatortisch beziehungsweise im Isolatorboden ausgebildet. Hierzu wird in einer ganz besonders bevorzugten Gestaltung der Isolatortisch beziehungsweise der Isolatorboden zur Ausbildung des Reservoirs nach unten hin ausgezogen, derart, dass das Reservoir hierdurch ausgebildet wird. Damit bildet besonders bevorzugt der nach unten hin ausgezogene Isolatorboden auch die Wände und den Boden des Reservoirs aus.

In einer bevorzugten Ausgestaltung wird die Integration des Reservoirs in den Isolatorraum so erreicht, dass der Isolatorraum von Isolatorwänden, Isolatordach, Isolatorboden und dem Reservoir begrenzt ist, bevorzugt auch von den Wänden und dem Boden des Reservoirs. Entsprechend sind Isolatorraum und Reservoir eins.

Durch den Isolatorboden wird bevorzugt eine Wanne beziehungsweise ein Gefäß ausgebildet. Das entsprechende Gefäß ist nach oben hin offen, so dass zum einen das Behandlungsfluid problemlos in das Reservoir eintreten kann und zum anderen eine problemlose Reinigung mittels der bereits im Isolator vorhandenen Reinigungsdüsen durchgeführt werden kann.

Weiterhin wird auf diese Weise eine vereinfachte Ausbildung des Reservoirs dahingehend erreicht, dass lediglich eine Verformung des Isolatorbodens beziehungsweise des Isolatortisches zur Ausbildung des Reservoirs vonnöten ist. Die separate Herstellung eines Gefäßes oder einer Wanne zur Ausbildung des Reservoirs ist entsprechend genauso wenig notwendig, wie die Bereitstellung einer aufwändigen Zuführ- beziehungsweise Abführverrohrung, welche eine Distanz zwischen einem separaten Reservoir und dem Isolatorraum überbrücken würde.

Bevorzugt ist das Reservoir als Gefäß oder Wanne ausgebildet, welches mit einer Reservoiröffnung versehen ist, welche mit dem Isolatorraum direkt verbunden ist und daher direkt mit dem Isolatorraum kommuniziert. Bevorzugt ist das Reservoir entsprechend als ein Gefäß oder eine Wanne ausgebildet, welches unterhalb des Isolators so angeordnet ist, dass die Behälteröffnung beispielsweise mit dem Isolatorboden beziehungsweise einem Isolatortisch abschließt, um die oben beschriebenen vorteilhaften Wirkungen zu erreichen.

In einer bevorzugten Weitergestaltung ist das Reservoir in dem Isolatorraum aufgenommen, bevorzugt vollständig in diesem aufgenommen. Auf diese Weise kann beispielsweise ein durch ein separates Gefäß ausgebildetes Reservoir in den Isolatorraum aufgenommen werden, beispielsweise in diesen hineingestellt werden, ohne dass eine Kontaminierung außerhalb des Isolatorraumes stattfinden kann. Das Reservoir ist in dieser Weitergestaltung vom Isolatorraum umschlossen.

Erfindungsgemäß weist der Isolatorraum Reinigungsdüsen zur Reinigung des Isolatorraumes auf, wobei die Reinigungsdüsen gleichzeitig auch zur Reinigung des Reservoirs dienen. So kann auf separate Reinigungsvorrichtungen zur Reinigung des Reservoirs verzichtet werden.

Bevorzugt ist der Isolatortisch beziehungsweise der Isolatorboden so in Richtung der Reservoiröffnung des Reservoirs hin geneigt, dass auf den Isolatortisch beziehungsweise den Isolatorboden auftreffendes Behandlungsfluid direkt in das Reservoir fließt.

Bevorzugt wird das Behandlungsfluid in einem Kreislauf geführt, dadurch, dass an einem tief gelegenen Punkt des Reservoirs das Behandlungsfluid abgezogen wird, zu einer Behandlungsdüse gefördert wird, und nach Behandlung des Behälters über den Isolatortisch beziehungsweise Isolatorboden wieder direkt in das Reservoir abfließt. Eine zusätzliche Vorrichtung zum Auffangen und Rücktransportieren des Behandlungsfluids zu dem Reservoir ist entsprechend nicht notwendig, sondern der ohnehin vorhandene Isolatorboden dient dazu, das verwendete Behandlungsfluid in das Reservoir zu leiten.

Bevorzugt sind in der Vorrichtung zur Behandlung von Behältern ein erstes und ein zweites Reservoir vorgesehen, wobei der jeweilige Isolatortisch beziehungsweise Isolatorboden so ausgebildet ist, dass das Behandlungsfluid aus einem ersten Bereich eines Isolatorraumes in das erste Reservoir abfließt, und von einem zweiten Bereich des Isolatorraumes in das zweite Reservoir abfließt. Auf diese Weise kann beispielsweise erreicht werden, dass das Desinfektionsmedium, mittels welchem die Behälter in einem ersten Bereich des Isolatorraumes, welcher auch als in sich abgeschlossener, eigenständiger Isolatorraum ausgebildet sein kann, behandelt wird, in das erste Reservoir abfließt, und Sterilwasser, mittels welchem die Behälter in einem zweiten Behandlungsschritt in einem weiteren Bereich des Isolatorraumes, beispielsweise auch in einem zweiten separaten Isolator, gespült wird, in das zweite Reservoir geleitet wird, um dort für eine mögliche weitere Verwendung zwischengespeichert zu werden.

### Kurze Beschreibung der Figuren

Bevorzugte weitere Ausführungsformen und Aspekte der vorliegenden Erfindung werden durch die nachfolgende Beschreibung der Figur näher erläutert. Dabei zeigt:

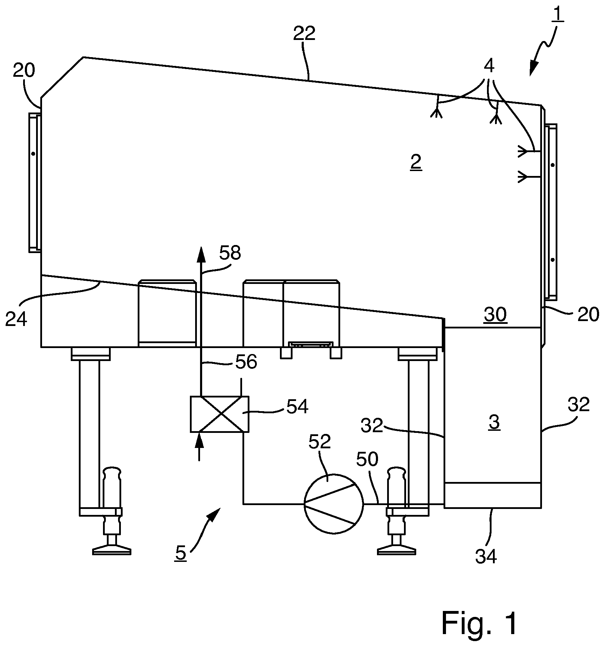

Figur 1 einen schematischen Querschnitt durch einen Isolatorraum mit einem Reservoir.

### Detaillierte Beschreibung bevorzugter Ausführungsbeispiele

Im Folgenden werden bevorzugte Ausführungsbeispiele anhand der Figur beschrieben.

Figur 1 zeigt eine Vorrichtung 1 zur Behandlung von Behältern in einer Getränkeabfüllanlage in Form einer Nass-Aseptik-Anlage, welche unter anderem einen Isolatorraum 2 aufweist, der durch schematisch angedeutete Isolatorwände 20, ein Isolatordach 22 sowie einen Isolatorboden 24 ausgebildet ist und gegenüber der Umgebung abgeschirmt ist. In dem Isolatorraum 2 findet die Behandlung der nicht gezeigten Behälter mittels in der schematischen Darstellung nur angedeuteter Behälterbehandlungsvorrichtungen 5 statt. Die Behälterbehandlungsvorrichtungen 5 können Sprüh- und/oder Rinsvorrichtungen zum Besprühen der Behälter von innen und von außen mit einem Desinfektionsmittel und zum nachfolgenden Spülen beziehungsweise Rinsen mit Sterilwasser umfassen. Weiterhin können in der schematischen Darstellung nicht gezeigte Transportvorrichtungen zum Transport der zu behandelnden Behälter durch den Isolatorraum 2 hindurch vorgesehen sein.

Ein Reservoir 3 zur Aufnahme des Behandlungsfluids, beispielsweise des Desinfektionsmittels oder des Sterilwassers, ist in Figur 1 schematisch gezeigt. Das Reservoir 3 ist in den Isolatorraum 2 integriert.

In dem in Figur 1 gezeigten Ausführungsbeispiel ist der Isolatorboden 24 nach unten hin ausgezogen, derart, dass das Reservoir 3 hierdurch ausgebildet wird. Der ausgezogene Isolatorboden 24 bildet so die Wände 32 und/oder den Boden 34 des Reservoirs 3 aus. Im gezeigten Ausführungsbeispiel ist der Isolatorboden 24 einstückig mit den Wänden 32 und dem Boden 34 des Reservoirs 3 ausgebildet.

In einer Variante können die Wände 32 und der Boden 34 des Reservoirs 3 aber auch durch separate Teile oder durch eine separate Wanne ausgebildet sein, welche mit dem Isolatorboden 24 und den Isolatorwänden 20 fest und gegenüber der Umgebung abschirmend verbunden ist.

Entsprechend wird der Isolatorraum 2 in dem gezeigten Ausführungsbeispiel durch die Isolatorwände 20, das Isolatordach 22, den Isolatorboden 24, die Wände 32 sowie den Boden 34 des Reservoirs 3 definiert. Diese Bereiche sind gegenüber der Umgebung fluiddicht und gasdicht ausgebildet, so dass ein Austreten von Kontaminierungen wie beispielsweise Desinfektionsdämpfe aus diesen Bereichen nicht möglich ist.

Das Reservoir 3 weist eine Reservoiröffnung 30 auf, welche direkt an den Isolatorraum 2 anschließt, beziehungsweise mit dem Isolatorraum 2 eine Einheit ausbildet.

Der Isolatorboden 24 ist weiterhin zumindest in den Bereichen, über welchen eine Behandlung der zu behandelnden Behälter mit dem Behandlungsfluid stattfindet, mit einer Neigung versehen und/oder als Schrägtisch ausgebildet. Damit fließt beispielsweise von den behandelten Behältern abfließendes und auf den Isolatorboden 24 auftreffendes Behandlungsfluid in Richtung des Reservoirs 3 beziehungsweise der Reservoiröffnung 30 ab.

In dem in Figur 1 gezeigten Ausführungsbeispiel ist, bei entleertem Reservoir 3, der Isolatorraum 2 durchgängig mit dem Reservoir 3 ausgebildet, derart, dass hier ein gemeinsamer integrierter Raum ausgebildet wird.

In dem Reservoir 3 kann entsprechend das Behandlungsfluid, beispielsweise das Desinfektionsmedium, aufgenommen werden, welches zur Behandlung der zu behandelnden Behälter verwendet wird und/oder welches während der Behandlung der zu behandelnden Behälter anfällt. Dadurch, dass das Reservoir 3 in den Isolatorraum 2 integriert ist, gelangen aus der Reservoiröffnung 30 austretende Desinfektionsmitteldämpfe, beispielsweise Chlordämpfe, direkt in den Isolatorraum 2, in welchem sie dann überwacht, behandelt oder zur Ableitung vorgesehen werden können.

Ein Austreten der entstehenden Kontaminierungen außerhalb des Isolatorraumes 2 wird dadurch verhindert, dass der Isolatorraum 2 gegenüber der Umgebung abgedichtet ist. Diese Abdichtung wird in bekannter Weise durch die Isolatorwände 20, das Isolatordach 22 sowie den Isolatorboden 24, welcher in dem gezeigten Ausführungsbeispiel zum Reservoir 3 hin ausgezogen ist, erreicht. In den Bereichen, in welchen beispielsweise Behälter in den Isolatorraum 2 eingeschleust oder aus diesem wieder ausgeschleust werden, sind entsprechende Schleusenvorrichtungen vorgesehen. Diese Schleusenvorrichtungen können in bekannter Weise beispielsweise in Form von Unterdruck/Überdruckanordnungen und leistungsstarken Absaugungen vorgesehen sein, mittels welchen die Druckverhältnisse und Luft-/Gasströmungen so eingestellt werden können, dass Kontaminierungen aus dem Isolatorraum 2 nicht in die Umgebung gelangen können.

Das Reservoir 3 bildet in dem Ausführungsbeispiel entsprechend einen Teil des Isolatorraumes 2 aus. In einer Alternative kann das Reservoir 3 auch durch ein separates Gefäß, beispielsweise eine Wanne, ausgebildet sein, welches beispielsweise in der gezeigten Art und Weise mit dem Isolatorraum 2 fluiddicht und gasdicht verbunden ist, so dass hier über diese direkte Verbindung eine Integration in den Isolatorraum 2 stattfindet. Eine entsprechende Reservoiröffnung 30 eines solchen Gefäßes ist dann ebenfalls direkt mit dem Isolatorraum 2 verbunden, so dass die oben beschriebenen Effekte erreicht werden können.

Durch die Integration des Reservoirs 3 in den Isolatorraum 2 kann auf eine eigene Überwachung des Reservoirs 3 sowie eine eigene hermetische Abdichtung des Reservoirs 3 gegenüber der Umgebung verzichtet werden, da sämtliche Emissionen des Reservoirs 3 in den Isolatorraum 2 hinein stattfinden. Der Isolatorraum 2 ist bei einer Vorrichtung 1 zur Behandlung von Behältern aber bereits auf die Handhabung und Weiterleitung der entsprechenden Emissionen vorbereitet, da diese auch im Behandlungsbetrieb, beispielsweise bei der Behälterdesinfektion, auftreten.

Der Isolatorraum 2 weist schematisch angedeutete Reinigungsdüsen 4 auf, welche zur Reinigung des Isolatorraumes 2 selbst beziehungsweise zur Reinigung der inneren Oberflächen der Isolatorwände 20, des Isolatordaches 22 sowie des Isolatorbodens 24 dienen. Solche Reinigungsdüsen 4 zur automatisierten Reinigung eines Isolatorraumes 2 von innen sind prinzipiell bekannt und werden beispielsweise in Nass-Aseptikanlagen verwendet.

Die Reinigungsdüsen 4 können im gezeigten Ausführungsbeispiel gleichzeitig auch zur Reinigung des Reservoirs 3 beziehungsweise der das Reservoir 3 begrenzenden Wände 32 sowie des Bodens 34 dienen. Eine separate Reinigungsvorrichtung mit den damit einhergehenden Steuervorrichtungen und Versorgungsleitungen zum Zuführen des Reinigungsmediums zur Reinigung des Reservoirs 3 ist damit nicht notwendig.

Die in der Figur 1 gezeigte Vorrichtung 1 ist entsprechend besonders zuverlässig ausgebildet, dahingehend, da eine zuverlässige Kontrolle und Überwachung der Emissionen aus dem Reservoir 3 erreicht werden kann. Dabei wird ein Austreten von schädlichen und/oder geruchsbelastenden Emissionen aus dem Isolatorraum 2 im Wesentlichen verhindert. Die von dem Reservoir 3 stammenden Emissionen werden durch die im und für den Isolatorraum 2 vorgesehenen Sicherheitsvorrichtungen kontrolliert und überwacht. Damit kann durch die Integration von Isolatorraum 2 und Reservoir 3 auf zusätzliche Sicherheitsvorkehrungen zur Überwachung des Reservoirs 3 verzichtet werden, so dass durch eine reduzierte Anzahl von Sicherheitsvorrichtungen auch deren Gesamtausfallwahrscheinlichkeit reduziert werden kann. Damit kann die Verfügbarkeit der Vorrichtung 1 insgesamt erhöht werden.

Um das in dem Reservoir 3 aufgenommene Behandlungsmedium der weiteren Behandlung zuzuführen, ist eine schematisch angedeutete Behälterbehandlungsvorrichtung 5 vorgesehen. Sie umfasst eine erste Rohrleitung 50, welche in einer unteren Ebene des Reservoirs 3 ansetzt und mittels welcher über eine Pumpe 52 das in dem Reservoir 3 aufgenommene Behandlungsfluid einem Wärmetauscher 54 zugeführt werden kann. Über eine entsprechende Zuleitung 56 wird das Behandlungsfluid dann direkt einer hier nur schematisch angedeuteten Behandlungsdüse 58 zur Beaufschlagung der zu behandelnden Behälter mit dem Behandlungsmedium zugeführt.

Durch die Behälterbehandlungsvorrichtung 5 wird es möglich, das in dem Reservoir aufgenommene Behandlungsfluid im Kreislauf zu führen. Entsprechend wird das Behandlungsfluid aus dem Reservoir 3 mittels der Behälterbehandlungsvorrichtung 5 an einer tieferen Ebene des Reservoirs 3 abgezogen, über den Wärmetauscher 54 entsprechend temperiert, und dann über die Behandlungsdüse 58 auf die zu behandelnden Behälter aufgebracht.

Durch die kurzen Rohrleitungen wird ein fast unmittelbares Beeinflussen der Temperierung und der Konzentration des Behandlungsfluids möglich, da entsprechende, nicht zu beeinflussende Behandlungsfluidvolumina in langen Rohrleitungen fortfallen.

Das von den behandelten Behältern wieder ablaufende Behandlungsfluid tropft dann auf den Isolatorboden 24, welcher aufgrund seiner Schrägstellung ein Einleiten des abtropfenden Behandlungsfluids über die Reservoiröffnung 30 in das Reservoir 3 zurück ermöglicht. Entsprechend kann auf eine einfache Weise ein Kreislauf hergestellt werden, mittels welchem das Behandlungsfluid auf die Behälter aufgebracht werden kann.

In einer bevorzugten Weiterbildung sind mindestens zwei Reservoirs vorgesehen, welche in nacheinander angeordneten Anlagenbereichen derart vorgesehen sind, dass beispielsweise in einem ersten Abschnitt des Isolatorraumes, beziehungsweise innerhalb eines ersten separaten Isolators, eine Behälterbehandlung durch Beaufschlagung der Behälter mit einem Desinfektionsmittel durchgeführt wird. Das Desinfektionsmittel fließt nach der Behälterbehandlung in das erste Reservoir. In einem nachfolgenden Abschnitt des Isolatorraumes beziehungsweise in einem weiteren separaten Isolator findet dann eine weitere Behälterbehandlung durch Spülen der vorher mit dem Desinfektionsmittel beaufschlagten Behälter mittels Sterilwassers statt. Das von den Behältern abfließende Sterilwasser wird durch den entsprechenden Isolatorboden aufgefangen und dem zweiten Reservoir zugeführt.

Auf diese Weise kann durch das Vorsehen der beiden in dem Isolatorraum integrierten Reservoirs ein effizienter Anlagenaufbau erreicht werden, welcher zum einen eine Reduktion des Platzverbrauches, zum anderen aber einen besonders zuverlässigen Betrieb der Anlage ermöglicht, welche in einem reduzierten Anlagenausfall und einer erhöhten Sicherheit der Behälterbehandlung resultiert.

Soweit anwendbar, können alle einzelnen Merkmale, die in den einzelnen Ausführungsbeispielen dargestellt sind, miteinander kombiniert und/oder ausgetauscht werden, ohne den Bereich der Erfindung zu verlassen.

### Bezugszeichenliste

- 1: Vorrichtung zur Behandlung von Behältern
- 2: Isolatorraum
- 20: Isolatorwand
- 22: Isolatordach
- 24: Isolatorboden
- 3: Reservoir
- 30: Reservoiröffnung
- 32: Wand
- 34: Boden
- 4: Reinigungsdüse
- 5: Behälterbehandlungsvorrichtung
- 50: erste Rohrleitung
- 52: Pumpe
- 54: Wärmetauscher
- 56: Zuleitung
- 58: Behandlungsdüse

## Patentansprüche

1. Vorrichtung (1) zur Desinfektion von Behältern in einer Getränkeabfüllanlage, umfassend eine Behälterbehandlungsvorrichtung (5) und einen gegenüber der Umgebung abgeschirmten Isolatorraum (2), in welchem eine Desinfektion von Behältern durchführbar ist, und ein Reservoir (3) zur Aufnahme eines Desinfektionsmittels zur Desinfektion der Behälter, wobei
das Reservoir (3) in den Isolatorraum (2) integriert ist und die Behälterbehandlungsvorrichtung (5) so vorgesehen ist, dass diese das Desinfektionsmittel in einer unteren Ebene des Reservoirs (3) abzieht und über eine Behälterbehandlungsdüse (58) auf die zu behandelnden Behälter aufbringt, und
der Isolatorraum (2) Reinigungsdüsen (4) zur Reinigung des Isolatorraumes (2) aufweist, und die Reinigungsdüsen (4) auch zur Reinigung des Reservoirs (3) ausgebildet sind.

2. Vorrichtung (1) gemäß Anspruch 1, **dadurch gekennzeichnet, dass** ein Teil des Isolatorraumes (2) das Reservoir (3) ausbildet.

3. Vorrichtung (1) gemäß Anspruch 1 oder 2, **dadurch gekennzeichnet, dass** der Isolatorraum nach unten durch einen Isolatortisch und/oder einen Isolatorboden (24) begrenzt ist und das Reservoir (3) im Isolatortisch beziehungsweise im Isolatorboden (24) ausgebildet ist.

4. Vorrichtung (1) gemäß Anspruch 3, **dadurch gekennzeichnet, dass** der Isolatorboden (24) zur Ausbildung des Reservoirs (3) nach unten hin ausgezogen ist.

5. Vorrichtung (1) gemäß Anspruch 3 oder 4, **dadurch gekennzeichnet, dass** der Isolatorboden (24) auch die Wände (32) und den Boden (34) des Reservoirs (3) ausbildet.

6. Vorrichtung (1) gemäß einem der vorstehenden Ansprüche, **dadurch gekennzeichnet, dass** der Isolatorraum (2) von Isolatorwänden (20), Isolatordach (22), Isolatorboden (24) und dem Reservoir (3) begrenzt ist, bevorzugt von den Wänden (32) und dem Boden (34) des Reservoirs (3).

7. Vorrichtung (1) gemäß einem der vorstehenden Ansprüche, **dadurch gekennzeichnet, dass** das Reservoir (3) in dem Isolatorraum (2) aufgenommen ist, bevorzugt vollständig in diesem aufgenommen ist.

8. Vorrichtung (1) gemäß einem der vorstehenden Ansprüche, **dadurch gekennzeichnet, dass** das Reservoir (3) als Gefäß, bevorzugt Wanne, mit einer Öffnung (30) vorgesehen ist, welche mit dem Isolatorraum (2) direkt verbunden ist.

9. Vorrichtung (1) gemäß einem der vorstehenden Ansprüche, **dadurch gekennzeichnet, dass** der Isolatortisch und/oder der Isolatorboden (24) so zu dem Reservoir (3), bevorzugt zu einer Reservoiröffnung (30), hin geneigt ist, das auf dem Isolatortisch beziehungsweise dem Isolatorboden (24) auftreffendes Desinfektionsmittel in das Reservoir (3) fließt.

10. Vorrichtung (1) gemäß einem der vorstehenden Ansprüche, **dadurch gekennzeichnet, dass** die Behälterbehandlungsvorrichtung (5) so vorgesehen ist, dass diese das Desinfektionsmitteld unter Druck und/oder temperiert über die Behälterbehandlungsdüse (58) auf die zu behandelnden Behälter aufbringt.

11. Vorrichtung (1) gemäß Anspruch 10, **dadurch gekennzeichnet, dass** ein Kreislauf für das Desinfektionsmittel ausgebildet ist.

12. Vorrichtung (1) gemäß einem der vorstehenden Ansprüche, **dadurch gekennzeichnet, dass** mindestens ein erstes Reservoir und ein zweites Reservoir vorhanden sind, wobei das erste Reservoir zur Aufnahme eines Desinfektionsmittels und das zweite Reservoir zur Aufnahme eines Spülmediums, insbesondere Sterilwasser, vorgesehen sind.

## Claims

1. Device (1) for disinfecting containers in a beverage bottling system, comprising a container treatment device (5) and an insulator space (2) shielded from the environment, in which a disinfection of containers can be carried out, and a reservoir (3) for receiving a disinfectant for the disinfection of the containers, wherein
the reservoir (3) is integrated into the insulator space (2) and the container treatment device (5) is provided so that said container treatment device draws off the disinfectant in a lower plane of the reservoir (3) and applies it by means of a container treatment nozzle (58) to the containers to be treated, and
the insulator space (2) has cleaning nozzles (4) for cleaning the insulator space (2), and the cleaning nozzles (4) are also designed for cleaning the reservoir (3).

2. Device (1) according to claim 1, **characterised in that** a part of the insulator space (2) forms the reservoir (3).

3. Device (1) according to claim 1 or 2, **characterised in that** the insulator space is delimited at the bottom by an insulator table and/or an insulator base (24) and the reservoir (3) is formed in the insulator table or in the insulator base (24).

4. Device (1) according to claim 3, **characterised in that** the insulator base (24) is extended downwards to form the reservoir (3).

5. Device (1) according to claim 3 or 4, **characterised in that** the insulator base (24) also forms the walls (32) and the base (34) of the reservoir (3).

6. Device (1) according to any one of the preceding claims, **characterised in that** the insulator space (2) is delimited by insulator walls (20), insulator roof (22), insulator base (24) and the reservoir (3), preferably by the walls (32) and the base (34) of the reservoir (3).

7. Device (1) according to any one of the preceding claims, **characterised in that** the reservoir (3) is accommodated in the insulator space (2), preferably is completely accommodated in said insulator space.

8. Device (1) according to any one of the preceding clams, **characterised in that** the reservoir (3) is provided as a vessel, preferably a trough, with an opening (30), which is connected directly to the insulator space (2).

9. Device (1) according to any one of the preceding claims, **characterised in that** the insulator table and/or the insulator base (24) is inclined towards the reservoir (3), preferably towards a reservoir opening (30), in such a manner that the disinfectant impinging on the insulator table or the insulator base (24) flows into the reservoir (3).

10. Device (1) according to any one of the preceding claims, **characterised in that** the container treatment device (5) is provided such that said container treatment device applies the disinfectant under pressure and/or tempered via the container treatment nozzle (58) to the containers to be treated.

11. Device (1) according to claim 10, **characterised in that** a circuit is formed for the disinfectant.

12. Device (1) according to any one of the preceding claims, **characterised in that** at least one first reservoir and a second reservoir are present, wherein the first reservoir is provided for receiving a disinfectant and the second reservoir is provided for receiving a flushing medium, in particular, sterile water.

## Revendications

1. Dispositif (1) de désinfection de récipients dans une installation de remplissage de boissons, comprenant un dispositif de traitement de récipients (5) et un espace d'isolement protégé de l'environnement (2), dans lequel une désinfection de récipients peut être réalisée, et un réservoir (3) pour recevoir un désinfectant pour désinfecter les récipients, dans lequel
le réservoir (3) est intégré dans l'espace d'isolement (2) et le dispositif de traitement de récipients (5) est prévu de telle sorte que celui-ci soutire le désinfectant dans un plan inférieur du réservoir (3) et l'applique par le biais d'une buse de traitement de récipients (58) sur les récipients à traiter, et
l'espace d'isolement (2) présente des buses de nettoyage (4) pour nettoyer l'espace d'isolement (2), et les buses de nettoyage (4) sont également formées pour nettoyer le réservoir (3).

2. Dispositif (1) selon la revendication 1, **caractérisé en ce qu'une** partie de l'espace d'isolement (2) forme le réservoir (3).

3. Dispositif (1) selon la revendication 1 ou 2, **caractérisé en ce que** l'espace d'isolement est limité vers le bas par une table d'isolement et/ou un plancher d'isolement (24) et le réservoir (3) est formé dans la table d'isolement ou dans le plancher d'isolement (24).

4. Dispositif (1) selon la revendication 3, **caractérisé en ce que** le plancher d'isolement (24) est retiré vers le bas pour former le réservoir (3).

5. Dispositif (1) selon la revendication 3 ou 4, **caractérisé en ce que** le plancher d'isolement (24) forme également les parois (32) et le plancher (34) du réservoir (3).

6. Dispositif (1) selon l'une quelconque des revendications précédentes, **caractérisé en ce que** l'espace d'isolement (2) est limité par des parois d'isolement (20), un toit d'isolement (22), un plancher d'isolement (24) et le réservoir (3), de préférence par les parois (32) et le plancher (34) du réservoir (3).

7. Dispositif (1) selon l'une quelconque des revendications précédentes, **caractérisé en ce que** le réservoir (3) est reçu dans l'espace d'isolement (2), de préférence est reçu complètement dans celui-ci.

8. Dispositif (1) selon l'une quelconque des revendications précédentes, **caractérisé en ce que** le réservoir (3) est prévu en tant que conteneur, de préférence en tant que cuve, ayant une ouverture (30) qui est reliée directement à l'espace d'isolement (2).

9. Dispositif (1) selon l'une quelconque des revendications précédentes, **caractérisé en ce que** la table d'isolement et/ou le plancher d'isolement (24) est incliné(e) vers le réservoir (3), de préférence vers une ouverture de réservoir (30), de telle sorte que le désinfectant arrivant sur la table d'isolement ou le plancher d'isolement (24) s'écoule dans le réservoir (3).

10. Dispositif (1) selon l'une quelconque des revendications précédentes, **caractérisé en ce que** le dispositif de traitement de récipients (5) est prévu de telle sorte que celui-ci applique le désinfectant sous pression et/ou tempéré par le biais de la buse de traitement de récipients (58) sur les récipients à traiter.

11. Dispositif (1) selon la revendication 10, **caractérisé en ce qu'**une circulation est formée pour le désinfectant.

12. Dispositif (1) selon l'une quelconque des revendications précédentes, **caractérisé en ce qu'**au moins un premier réservoir et un second réservoir sont présents, dans lequel le premier réservoir est prévu pour recevoir un désinfectant et le second réservoir est prévu pour recevoir un moyen de rinçage, en particulier de l'eau stérile.
